# EUROPEAN PATENT SPECIFICATION

(11) **EP 0 688 203 B1**
(45) Date of publication and mention of the grant of the patent: **23.10.1996**
(21) Application number: 94910423.6
(22) Date of filing: 11.03.1994
(51) Int. Cl.: A61K 7/42

(54) **COSMETIC COMPOSITION FOR ARTIFICIAL TANNING OF THE SKIN**
KOSMETISCHES KÜNSTLICHES HAUTBRÄUNUNGSMITTEL
COMPOSITION COSMETIQUE POUR LE BRONZAGE ARTIFICIEL DE LA PEAU

(30) Priority: 13.03.1993 GB 9305183
(43) Date of publication of application: 27.12.1995
(73) Proprietor: The Boots Company PLC, Nottingham, Nottinghamshire NG2 3AA (GB)
(72) Inventor: LINNINGTON, Helen Louise, Nottingham NG2 3AA (GB); SHEARD, Christine, Nottingham NG2 3AA (GB)
(74) Representative: Kirk, Martin John
(86) International application number: EP9400815
(87) International publication number: WO9421221

(56) References cited:
- EP-A- 0 425 324
- SEIFEN, öLE, FETTE, WACHSE vol. 16 , 1972 page 512

## Description

The present invention relates to compositions that produce tanned skin in the absence of UV radiation in particular to those comprising dihydroxyacetone as the tanning agent.

Known artificial tanning compositions use the artificial tanning agent dihydroxyacetone of the following formula:- 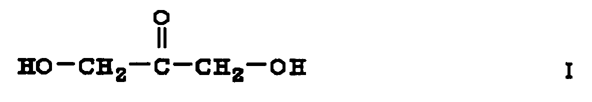 (referred to hereinafter as 'DHA'). The term 'artificial tanning agent' is used hereinafter to denote an agent which acts on the skin to produce a skin tan without requiring ultra-violet radiation (UV). This is in contrast with agents that act to accelerate the natural tanning processes that occur when skin is exposed to UV.

DHA reacts with the amino acids and amino groups of keratin proteins present in the outer layers of the skin epidermis, to form brown-coloured compounds. The reaction is believed to be similar to the Malliard reaction (non-enzymic browning) in which reducing sugars react with amino acids, peptides and proteins to form compounds containing amino and carbonyl functionality, these compounds being polymerised to brown melanoidines.

Artificial tanning agents have the advantage of producing a skin tan without the need to expose the skin to UV radiation. The increasing tendency of people to sunbathe by exposing skin for long periods to UV radiation from sunlight or sunlamps in order to produce a skin tan, has led to an increase in the incidence of skin cancer worldwide, particularly amongst fair skinned people. Depletion of atmospheric ozone has also increased the intensity of UV radiation reaching the earth particularly at higher latitudes. Thus, the use of artificial tanning agents to produce tanned skin is increasingly advantageous, not only in less sunny climates, but in sunny climates as an alternative or supplement to exposure of the skin to UV radiation.

Current artificial tanning compositions comprising DHA are not completely satisfactory. The skin colour produced by a DHA composition is a yellow-brown which can look unnatural especially on fair skin. As DHA takes several hours to develop colour on the skin, it can be difficult to apply the DHA tanning composition evenly over the skin, which can result in a streaky appearance. The colour cannot be washed off, but fades as the upper layers of the epidermis wear away.

Seifen, öLe, Fette, Wachse, vol. 16, 1972, page 512 discloses use of a conventional iron oxide pigment with DHA. The well known iron oxide pigment is simply used to shade temporarily the colour of the skin tan produced by the DHA. This document does not disclose that iron salts may act directly with DHA to alter the tone of the artificial tan permenantly created by the DHA in the skin epidermis.

Japanese patent application J61 27367 (Sunstar) discloses a composition for dying the skin proposed as an alternative to a DHA composition. A ferrous salt is used (e.g. an alternative to infra-red radiation) to catalyse diol metabolites of dopa, dopomia, noradrenalin and/or adreralin (not DHA) to dye the skin. The ferrous salt is used as a catalyst and does not alter the skin tone of the artificial tan. This application teaches away from use of iron salts and DHA as the tanning compositions disclosed have a different tanning mechanism than the Maillard reaction used by DHA. There would therefore be no motivation from Sunstar for a person skilled in the art to add iron salts to DHA compositions.

EP 425324 (L'Oreal) discloses DHA compositions with indole derivatives to produce a more natural looking tan. There is no disclosure of using iron salts in these compositions.

It is an object of the invention to provide an artificial tanning product comprising DHA, which produces a more natural colour, that is a less yellow coloured, artificial tan, and which develops colour more rapidly so as to be more easy to apply as an even coat on the skin to avoid streaking, compared to known artificial tanning products comprising DHA.

It has been discovered that in the presence of iron salts, DHA produces an artificial tan less yellow in colour. The mechanism of action may be formation of complexes between iron and DHA (or its reaction products) acting as a ligand. The presence of iron also causes an immediate pale colour to be produced when the DHA is initially applied to the skin, thus aiding application in an even layer to avoid a streaky appearance. After a few hours this pale colour darkens to give the final darker colour.

The present invention comprises an artificial tanning product comprising one or more compositions containing, separately or together, a cosmetically effective amount of dihydroxy acetone as an artificial tanning agent and one or more iron salt or salts with the proviso that the iron salt or salts are other than iron oxide. II the product comprises two or more compositions these may be packaged in a single pack comprising two or more parts.

The amount of DHA present in one or more composition or compositions comprising the present invention may be formulated to suit light, medium or dark skin types, preferably in an amount from about 0.1% to about 10%, more preferably from about 1% to about 7%, most preferably from about 2% to about 5% by weight of that composition.

The iron salt or salts can be any ferrous or ferric iron salt for example nitrate, gluconate or citrate. Preferably, the iron salt or salts are present in one or more of the composition or compositions of the product of the invention in an amount from about 0.1% to about 20%, more preferably from about 0.5% to about 10%, most preferably from about 1% to about 5% by weight of that composition. Particularly good results are obtained when using ferric salts in combination with a DHA tanning composition, to produce an artificial tan of less yellow tone, than produced by a DHA tanning composition alone.

Sunscreening agents may be incorporated into any of the composition or compositions comprising the present invention. The term 'sunscreening agent' is used herein to encompass any ingredient or ingredients used in tanning lotions, sunscreens and sunblockers or the like, which are intended for use on the body to provide protection against UV radiation produced from the sun or other artificial sources. Examples of suitable sunscreening agents include:-
a) titanium dioxide;
b) p-aminobenzoic acids, esters and derivatives thereof, such as 2-ethylhexyl p-dimethylaminobenzoate;
c) methoxycinnamate esters such as 2-ethylhexyl p-methoxycinnamate, 2-ethoxyethyl p-methoxycinnamate or α, β-di-(p-methoxycinnamoyl)-α'-(2-ethylhexanoyl) glycerin;
d) benzophenones such as oxybenzone;
e) dibenzoylmethanes;
f) salicylate esters;
g) zinc oxide; and
h) mixtures thereof.

Any sunscreening agent may be present in any of the composition or compositions comprising the present invention in an amount from about 0.1% to about 20% by weight of that composition.

Any of the composition or compositions comprising the present invention may be a gel.

Any of the composition or compositions comprising the present invention may include either oil-in-water or water-in-oil emulsions. The oil phase of emulsions comprising the present invention may include for example:
a) hydrocarbon oils such as paraffin or mineral oils;
b) waxes such as beeswax or paraffin wax;
c) natural oils such as sunflower oil, apricot kernel oil, shea butter of jojoba oil;
d) silicone oils such as dimethicone, cyclomethicone or cetyldimethicone;
e) fatty acid esters such as isopropyl palmitate or isopropyl myristate;
f) fatty alcohols such as cetyl alcohol or stearyl alcohol; and
g) mixtures thereof.

In the preferred oil-in-water composition or compositions comprising the present invention, the oil phase comprises from about 5% to about 30%, more preferably from about 10% to about 20%, by weight of that composition.

Emulsifiers that may be used in any of the composition or compositions comprising the present invention may be any emulsifiers known in the art for use in water-in-oil or oil-in-water emulsions. Water-in-oil and oil-in-water compositions can be prepared by using an emulsifier or mixture of emulsifiers selected from known cosmetically acceptable emulsifiers which include:
a) sesquioleates such as the sorbitan sesquioleate which is available commercially from ICI under the trade name Arlacel 83;
b) ethoxylated esters of derivatives of natural oils such as the polyethoxylated ester of hydrogenated castor oil which is available commercially from ICI under the trade name Arlacel 989;
c) silicone emulsifiers such as the silicone polyols which are available commercially from Th. Goldschmidt AG under the trade name ABIL WS05 and from Dow Corning under the trade designation Silicone Fluid 3225C;
d) fatty acid soaps such as potassium stearate;
e) ethoxylated fatty alcohols, such as those which are available commercially from ICI under the trade name Brij and from Croda under the trade name Cithrol GMS A/S;
f) sorbitan esters, such as those which are available commercially from Croda under the trade name Crill;
g) ethoxylated sorbitan esters, such as those which are available commercially from ICI under the trade name Tween;
h) ethoxylated fatty acid esters such as the ethoxylated stearates, which are available commercially from ICI under the trade name Myrj;
i) ethoxylated mono, di, and triglycerides, such as those which are available commercially from Alfa Chemicals under the trade name Labrafil;
j) ethoxylated fatty acids, such as those which are available commercially from Alfa Chemicals under the trade name Tefose;
k) anionic emulsifiers such as anionic esters of mono and di glycerides available commercially from Grindsted Products Ltd under the trade name Grindtek CA-P (which is not included in any composition or compositions that contain an iron salt or salts); and
l) mixtures thereof.

The amount of emulsifier optionally present in the water-in-oil compositions or compositions comprising the present invention is preferably in the range from about 0.1% to about 20% by weight of that composition. The amount of emulsifier optionally present in the oil-in-water composition or compositions comprising the present invention is preferably in the range from about 0.1% to about 20% by weight of that composition.

Any of the composition or compositions comprising the present invention may additionally include one or more other component or components which will be well known to those skilled in the art, for example:
a) emulsion stabilisers such as stearyl alcohols or cetyl alcohol, or emulsion stabilising salts such as sodium chloride, sodium citrate or magnesium sulphate, preferably in an amount from about 0.1% to about 5% by weight of that composition;
b) humectants such as glycerin or 1,3 butylene glycol, preferably in an amount from about 0.1% to about 30% by weight of that composition;
c) thickening agents such as hydroxyethyl cellulose or the acrylic acid polymer available commercially from Honeywill and Stein Ltd under the trade name Junlon PW110, preferably in an amount from a trace amount to about 1% by weight of that composition;
d) sequestrants such as the tetra sodium ethylene diamine tetra acetate dihydrate available commercially from Rhône Poulenc under the tradename Sequestene NA4, preferably in an amount from a trace amount to about 1% by weight of that composition, sequestrants not being included in any composition or compositions that contain an iron salt or salts;
e) anti-oxidants such as α tocopherol, DLα tocopherol acetate or butylated hydroxytoulene, preferably in an amount from a trace amount to about 2%, more preferably from a trace amount to about 1%, by weight of that composition;
f) emollients such as mineral oil, polymethylsiloxane, sweet almond oil, petroleum jelly, isopropyl myristate or triglycerides of fatty acids (for example lauric triglyceride, capric/caprylic triglyceride, or the mixed triglycerides available commercially from Huls UK Ltd under the trade name Miglyol 810), preferably in an amount from about 0.1% to about 30% by weight of that composition;
g) moisturisers such as D-panthenol, preferably in an amount from a trace amount to about 1% by weight of that composition;
h) agents for adjusting pH such as sodium citrate, potassium hydroxide or citric acid monohydrate, preferably in an amount from a trace amount to about 1% by weight of that composition;
i) film formers to assist spreading on the surface of the skin, such as alkylated polyvinylpyrrolidones, preferably in an amount from a trace amount to about 1% by weight of that composition;
j) preservatives such as bronopol, sodium dehydroacetate, isothiazolone or diazolidinylurea, preferably in an amount from a trace amount to about 1% by weight of that composition;
k) perfumes and colouring; and
l) mixtures thereof.

The present invention further provides a method of applying an artificial tanning product to an area of skin comprising the steps of:
a) applying to the skin area a composition comprising one or more iron salt or salts;
b) applying to the skin area a tanning composition comprising a cosmetically effective amount of dihydroxyacetone as an artificial tanning agent.

The steps of the method of the present invention may be performed substantially simultaneously, or sequentially in any order. If the steps are substantially simultaneous, then the iron salt composition and the tanning composition may be a single composition. If the steps are sequential then it is particularly preferred that the iron salt composition is applied prior to the tanning composition. In this preferred method, the iron salt composition advantageously comprises a moisturiser as it has been found that application of a moisturiser prior to application of the tanning composition aids production of an even skin colour. The iron salt composition used in the method of the present invention may comprise any composition comprising an iron salt or salts described herein. Similarly, the tanning composition used in the method of the present invention, may comprise any composition comprising DHA described herein.

The invention will now be illustrated by the following examples. Where results for a control experiment are also listed in an example, this control experiment was carried out identically to the procedure indicated below for that example using the same proportions of the same ingredients, in the composition or compositions of that example except that the iron salt or salts were removed.

Various trade names and the like known to those skilled in the art are used to describe ingredients used in the compositions listed below. For convenience the chemical composition of these ingredients will now be described.
Steareth 2 is a polyethylene glycol stearyl alcohol ether having an average of 2 ethylene oxide units per molecule available commercially from ICI under the trade mark Brij 72®;
Steareth 21 is a polyethylene glycol stearyl alcohol ether having an average of 21 ethylene oxide units per molecule available commercially from ICI under the trade mark Brij 721® ;
Grindtek CA-P is a hydrogenerated palm oil glyceride available commercially from Grinsted Products Ltd; Miglycol 812 is a mixed triglyceride of standard C₈ to C₁₂ acids available commercially from Huls UK Ltd;
Silicone fluid F111/300 is a polydimethylsiloxane available commercially from Dow Corning;
Junlon PW110 is an acrylic acid polymer available commercially from Honeywill and Stein Ltd;
Sequestrene NA4 is a tetra sodium ethylene diamine tetra acetate dihydrate available commercially from Rhône Poulenc;
PEG 20 stearate is a polyethylene glycol stearate having an average of 20 ethylene oxide units per molecule;
PEG 7 glycerol cocoate is a polyethylene glycol glycerol cocoate having an average of 7 ethylene oxide units per molecule;
Ceteth 20 is a polyethylene glycol cetyl alcohol ether having an average of 20 ethylene oxide units per molecule;
Silicone fluid 20cs is a silicone polyol emulsifier; and Silicone fluid 1000cs is a silicone polyol emulsifier.

### Example 1

### Compositions

### A) Iron salt composition

An iron salt composition for use as a pre-moisturiser containing the following ingredients was prepared as outlined below:-

| Ingredient | % w/w |
|---|---|
| Ferrous gluconate (DHA enhancer) | 2.0 |
| Hydroxy ethyl cellulose (thickener) | 0.25 |
| Glycerol (humectant) | 4.0 |
| 1,3 Butylene glycol (humectant) | 4.0 |
| Steareth 2 (water-in-oil emulsifier) | 2.0 |
| Steareth 21 (oil-in-water emulsifier) | 1.0 |
| Light liquid paraffin (emollient) | 10.0 |
| Polydimethylsiloxane (emollient) | 2.0 |
| Stearyl alcohol (emulsion stabiliser) | 2.3 |
| Glyceryl monostearin (water-in-oil emulsifier) | 3.0 |
| DLα tocopherol acetate (anti-oxidant) | qs |
| Preservative | qs |
| Perfume | qs |
| Purified water | to 100 |

The water soluble ingredients are mixed together and heated to 70°C. The thickener is added to the water phase ingredients using a high shear mixer, followed by the iron salt. The oil soluble ingredients are combined in a separate vessel, heated to 70°C and then added to the water phase mixture using a high shear mixer. The combined mixture is then mixed using the high shear mixer for a further 10 minutes, after which the mixture is allowed to cool to room temperature and preservative and perfume were added as desired.

### B) DHA-tanning composition

An artificial tanning cream comprising DHA to be used in conjunction with the iron composition prepared above was prepared from the following ingredients in the method described below:-

| Ingredient | % w/w |
|---|---|
| Dihydroxyacetone (tanning agent) | 4 |
| Grindtek CA-P (oil-in-water emulsifier) | 2.5 |
| Miglyol 812 (emollient) | 3 |
| DL-α tocopherol acetate (anti-oxidant) | 0.2 |
| Almond oil (emollient) | 2.0 |
| Glycerin BP (humectant) | 4.0 |
| Silicone Fluid F111/300 (emollient) | 1 |
| Light liquid paraffin (emollient) | 3.5 |
| Cetyl Alcohol (emulsion stabiliser) | 1 |
| Junlon PW110 (thickener) | 0.1 |
| White soft paraffin BP (emollient) | 1.5 |
| Sodium citrate (pH adjusting agent) | 0.25 |
| Sequestrene NA4 (sequestrant) | 0.02 |
| Potassium hydroxide (pH adjusting agent) | 0.02 |
| Citric Acid (pH adjusting agent) | 0.01 |
| Butylated hydroxy toluene (anti-oxidant) | 0.01 |
| Perfume | 0.29 |
| Preservative | qs |
| Purified water | to 100 |

The water soluble ingredients are mixed together and heated to 70°C. The thickening agent is added to the water phase ingredients using a high shear mixer followed by the pH adjusting agents to keep the pH to about 3. The oil soluble ingredients are combined in a separate vessel, heated to 70°C and then added to the water phase mixture using a high shear mixer. The combined mixture is mixed using the high shear mixer for a further 10 minutes, after which the mixture is allowed to cool to room temperature. The DHA was added (and preservative if desired) and the pH of the mixture was adjusted if necessary.

### Results

Both colour and intensity of the skin of human volunteers before and after treatment were measured using a Minolta Chromameter (Model CR-200) as below.

A square test area (40 mm x 40 mm) was marked on the forearm of a volunteer and the surface reflectance of that area was measured using the Minolta Chromameter. 40µl of the iron composition above was then applied to the test area and rubbed into the skin with a glass rod and left for 5 minutes to dry. Then 40µl of the tanning composition above was rubbed into the test area in a similar manner and left to dry for 10 minutes, after which the surface reflectance of the test area was measured again. The amount of light reflected, which is detected by the chromameter, gives a quantitative indication of skin colour, which is recorded as 'L', 'a' and 'b' values.

The 'L' value gives a quantitative indication of the darkness of the skin, with a lower value indicating darker.

The 'a' value gives a quantitative indication of the amount of red or green present in the skin, with a higher value indicating more red.

The 'b' value gives a quantitative indication of the amount of blue or yellow present in the skin with a higher value indicating more yellow.

| | 'L' value | 'a' value | 'b' value |
|---|---|---|---|
| Example 1 | 67.9 | 6.5 | 17.9 |
| Control | 67.5 | 7.7 | 17.5 |

### Example 2

### Compositions

Iron salt composition and DHA tanning composition were prepared and used as described in Example 1, with the exception that the iron salt composition contained 2% of ferric nitrate by weight of the composition instead of 2% of ferrous gluconate.

### Results

The results of the Minolta colour test are tabulated below:

| | 'L' value | 'a' value | 'b' value |
|---|---|---|---|
| Example 2 | 65.3 | 9.1 | 13.6 |
| Control | 65.8 | 8.9 | 15.0 |

### Examples 3 and 4

Iron salt compositions and DHA tanning compositions were prepared as described in Examples 1 and 2 respectively, with the addition of 2% w/w α-tocopherol as an anti-oxidant/deodorant to each composition. The compositions of Examples 3 and 4 when used as described in Example 1 produced a similar improvement in tan colour and intensity compared to control compositions as described above for Examples 1 and 2.

Some other formulations which may be used as DHA tanning compositions of the present invention are given below:

### I) Self Tan Cream 1

| Ingredient | % w/w |
|---|---|
| Dihydroxyacetone (tanning agent) | 4.0 |
| Octyl Methoxycinnamate (sunscreening agent) | 1.0 |
| PEG 20 Stearate (emulsifier) | 1.3 |
| Cetearyl alcohol (emulsion stabiliser) | 2.3 |
| Cetyl alcohol (emulsion stabiliser) | 1.0 |
| Liquid Paraffin (emollient) | 1.6 |
| Preservative and Perfume | qs |
| Purified water | to 100 |

### II) Self Tan Cream 2

| Ingredient | % w/w |
|---|---|
| Dihydroxyacetone (tanning agent) | 4.0 |
| Polyvinylpyrrolidone (PVP) Copolymer (film former) | 2.0 |
| Mixed Triglycerides (emollient) | 4.0 |
| 1,3 Butylene Glycol (humectant) | 5.0 |
| Ethoxylated Stearyl Alcohol (emulsifier) | 1.5 |
| Cetearyl alcohol (emulsion stabiliser) | 2.5 |
| PEG 7 Glyceryl Cocoate (emulsion stabiliser) | 1.5 |
| Xanthan Gum (emollient) | 0.3 |
| Glycerol (humectant) | 2.5 |
| Carboxymethyl Cellulose (thickener) | 0.4 |
| White Soft Paraffin (emollient) | 2.0 |
| Ceteth 20 (emulsifier) | 0.9 |
| Cocoamidopropyl betaine (emulsifier) | 2.0 |
| Preservative, Colour and Perfume | qs |
| Purified water | to 100 |

### III) Tan Maintainer

| Ingredient | % w/w |
|---|---|
| Dihydroxyacetone (tanning agent) | 1.2 |
| Alpha bisabolol (anti-phlogistic) | 0.1 |
| Glycerate/methacrylic acid polymer (emollient) | 2.0 |
| D Panthenol (moisturiser) | 0.6 |
| Cetearyl Isononate (emulsifier) | 2.0 |
| Hydrogenated Palm Oil Glycerides Citrate | 2.0 |
| (emulsifier) | |
| Silicone Fluid 20cs (emollient) | 2.0 |
| 1,3 Butylene Glycol (humectant) | 4.0 |
| Arachidyl Propionate (emulsifier) | 1.0 |
| Cetyl alcohol (emulsion stabiliser) | 2.0 |
| Theobroma Oil (emollient) | 1.0 |
| Acrylic Acid Copolymer (thickener) | 0.2 |
| Liquid Paraffin (emollient) | 4.6 |
| Allantoin (dermatological agent) | 0.2 |
| White Soft Paraffin (emollient) | 2.0 |
| Glycerol (humectant) | 0.8 |
| Potassium Hydroxide (pH adjuster) | 0.07 |
| Preservative, Colour and Perfume | qs |
| Purified water | to 100 |

### IV) Sunscreen with DHA

| Ingredients | % w/w |
|---|---|
| Dihydroxyacetone (tanning agent) | 0.5 |
| Butyl methoxydibenzoyl methane (sunscreening agent) | 3.0 |
| Octyl methoxycinnamate (sunscreening agent) | 7.3 |
| Benzophenone (sunscreening agent) | 2.0 |
| Cetearyl Isononate (emulsifier) | 9.2 |
| Glycerine esters (humectant) | 5.4 |
| Microcrystalline wax (thickener) | 3.0 |
| Acrylic acid copolymer (thickener) | 0.5 |
| Silicone Fluid 1000cs (emollient) | 5.0 |
| 1,3 Butylene Glycol (humectant) | 4.9 |
| Cetyl Alcohol (emulsion stabiliser) | 1.5 |
| Ceteareth Stearate (emulsifier) | 1.0 |
| Almond Oil (emollient) | 0.5 |
| Tetrahydroxypropyl ethylenediamine (sequestrant) | 1.2 |
| Preservative, Perfume and Antioxidant | qs |
| Purified water | to 100 |

### V) Sun Lotion with DHA

| Ingredients | % w/w |
|---|---|
| Dihydroxyacetone (tanning agent) | 0.5 |
| Butyl Methoxydibenzoyl methane (sunscreening agent) | 1.2 |
| Benzophenone (sunscreening agent) | 2.0 |
| Cetearyl Isononate (emulsifier) | 9.7 |
| Glycerine esters (humectant) | 5.4 |
| Silicone Fluid 1000cs (emollient) | 5.0 |
| Microcrystalline wax (thickener) | 2.0 |
| 1,3 Butylene Glycol (humectant) | 4.9 |
| Cetyl alcohol (emulsion stabiliser) | 0.3 |
| Ceteareth stearate (emulsifier) | 1.0 |
| Acrylic acid copolymer (thickener) | 0.4 |
| Liquid Paraffin (emollient) | 8.0 |
| Tetrahydroxypropyl ethylenediamine (sequestrant) | 1.0 |
| Almond Oil (emollient) | 0.5 |
| Preservative, Perfume and Antioxidant | qs |
| Purified water | to 100 |

## Claims

1. An artificial tanning product comprising one or more compositions containing, separately or together, a cosmetically effective amount of dihydroxyacetone as an artificial tanning agent, and one or more iron salt or salts, with the proviso that the iron salt or salts are other than iron oxide.

2. A product as claimed in claim 1, in which the iron salt or salts are present in one or more of the compositions in an amount from about 0.1% to about 20% by weight of that composition.

3. A product as claimed in either preceding claim, in which the iron salt or salts are present in one or more of the compositions in an amount from about 0.5% to about 10% by weight of that composition.

4. A product as claimed in any preceding claim, in which the iron salt or salts are present in one or more of the compositions in an amount from about 1% to about 5% by weight of that composition.

5. A product as claimed in any preceding claim, in which the iron salt or salts are selected from nitrate, gluconate and citrate.

6. A product as claimed in claim 5, in which the iron salt or salts are ferric nitrate and/or ferric citrate.

7. A product as claimed in any preceding claim, which comprises a single composition.

8. A product as claimed in any of claims 1-6, in which comprises two or more compositions packaged in the form of a pack of two or more parts.

9. A product as claimed in any of claims 1 to 8, which one or more of the compositions comprising an iron salt or salt further comprises a moisturiser.

10. A method of application of an artificial tanning product to an area of skin comprising the steps of:-
(a) applying to the skin area, a composition comprising one or more iron salt or salts with the proviso that the iron salt or salts are other than iron oxide;
(b) applying to the skin area a tanning composition comprising a cosmetically effective amount of dihydroxyacetone as an artificial tanning agent.

11. A method as claimed in claim 11, in which the iron salt composition comprises any composition comprising iron salt or salts which is described in any of claims 1 to 10.

12. A method as claimed in either of claims 11 or 12, in which the tanning composition comprises any composition comprising dihydroxyacetone which is described in any of claims 1 to 10.

13. A method as claimed in any of claims 11 to 13, in which the tanning composition is applied after the iron salt composition.

14. A method as claimed in any of claims 11 to 13, in which the tanning composition and iron salt composition are applied substantially simultaneously.

15. A method as claimed in claim 15, in which the iron salt composition and the tanning composition together form a single composition.

## Patentansprüche

1. Künstliches Hautbräunungsmittel, enthaltend eine oder mehrere Zusammensetzungen, die getrennt oder zusammen eine kosmetisch wirksame Menge Dihydroxyaceton als künstliches Hautbräunungsmittel und ein oder mehrere Eisensalze enthalten, mit der Maßgabe, daß das oder die Eisensalze kein Eisenoxid sind.

2. Produkt nach Anspruch 1, in dem das oder die Eisensalze in einer oder mehreren der Zusammensetzungen in einer Menge von etwa 0,1 bis etwa 20 Gew.-% dieser Zusammensetzung vorhanden sind.

3. Produkt nach einem der vorstehenden Ansprüche, in dem das oder die Eisensalze in einer oder mehreren der Zusammensetzungen in einer Menge von etwa 0,5 bis etwa 10 Gew.-% dieser Zusammensetzung vorhanden sind.

4. Produkt nach einem der vorstehenden Ansprüche, in dem das oder die Eisensalze in einer oder mehreren der Zusammensetzungen in einer Menge von etwa 1 bis etwa 5 Gew.-% dieser Zusammensetzung vorhanden sind.

5. Produkt nach einem der vorstehenden Ansprüche, in dem das oder die Eisensalze aus Nitrat, Gluconat und Citrat ausgewählt sind.

6. Produkt nach Anspruch 5, in dem das oder die Eisensalze Eisen(III)-nitrat und/oder Eisen(III)-citrat sind.

7. Produkt nach einem der vorstehenden Ansprüche, das eine einzige Zusammensetzung enthält.

8. Produkt nach einem der Ansprüche 1 bis 6, das zwei oder mehrere in Form einer Packung aus zwei oder mehr Bestandteilen abgepackte Zusammensetzungen enthält.

9. Produkt nach einem der Ansprüche 1 bis 8, in dem eine oder mehrere der ein oder mehrere Eisensalze enthaltende Zusammensetzungen außerdem ein feuchtigkeitsspendendes Mittel enthält.

10. Verfahren zur Anwendung eines künstlichen Bräunungsmittels auf einen Hautbereich mit folgenden Schritten:
(a) Aufbringen einer Zusammensetzung, die ein oder mehrere Eisensalze enthält, bei dem bzw. denen es sich jedoch nicht um Eisenoxid handeln darf, auf den Hautbereich;
(b) Aufbringen einer Bräunungszusammensetzung, die eine kosmetisch wirksame Menge Dihydroxyaceton als künstliches Bräunungsmittel enthält, auf den Hautbereich.

11. Verfahren nach Anspruch 10, in dem die Eisensalzzusammensetzung jede beliebige Zusammensetzung enthält, die ein oder mehrere Eisensalze wie in Anspruch 1 bis 9 beschrieben aufweist.

12. Verfahren nach Anspruch 10 oder 11, in dem die Bräunungszusammensetzung jede beliebige Dihydroxyaceton enthaltende Zusammensetzung wie in Anspruch 1 bis 9 beschrieben aufweist.

13. Verfahren nach einem der Ansprüche 10 bis 12, in dem die Bräunungszusammensetzung nach der Eisensalzzusammensetzung aufgebracht wird.

14. Verfahren nach einem der Ansprüche 10 bis 12, bei dem die Bräunungszusammensetzung und die Eisensalzzusammensetzung im wesentlichen gleichzeitig aufgebracht werden.

15. Verfahren nach Anspruch 14, in dem die Eisensalzzusammensetzung und die Bräunungszusammensetzung gemeinsam eine einzige Zusammensetzung bilden.

## Revendications

1. Produit de bronzage artificiel comprenant une ou plusieurs compositions renfermant, séparément ou conjointement, une quantité cosmétiquement efficace de dihydroxyacétone servant d'agent de bronzage artificiel, et un ou plusieurs sels de fer, sous réserve que le ou les sels de fer soient des sels autres que l'oxyde de fer.

2. Produit suivant la revendication 1, dans lequel le ou les sels de fer sont présents dans une ou plusieurs des compositions en une quantité d'environ 0,1 % à environ 20 % en poids d'une telle composition.

3. Produit suivant l'une des revendications précédentes, dans lequel le ou les sels de fer sont présents dans une ou plusieurs des compositions en une quantité d'environ 0,5 % à environ 10 % en poids d'une telle composition.

4. Produit suivant l'une quelconque des revendications précédentes, dans lequel le ou les sels de fer sont présents dans une ou plusieurs des compositions en une quantité d'environ 1 % à environ 5 % en poids d'une telle composition.

5. Produit suivant l'une quelconque des revendications précédentes, dans lequel le ou les sels de fer sont choisis entre un nitrate, un gluconate et un citrate.

6. Produit suivant la revendication 5, dans lequel le ou les sels de fer consistent en nitrate ferrique et/ou citrate ferrique.

7. Produit suivant l'une quelconque des revendications précédentes, qui comprend une seule composition.

8. Produit suivant l'une quelconque des revendications 1 à 6, qui comprend deux ou plus de deux compositions conditionnées dans un emballage en deux ou plus de deux parties.

9. Produit suivant l'une quelconque des revendications 1 à 8, dans lequel une ou plusieurs des compositions renfermant un ou plusieurs sels de fer comprennent en outre un agent d'humectation.

10. Procédé d'application d'un produit de bronzage artificiel à une surface de la peau, comprenant les étapes :
(a) d'application à la surface de la peau d'une composition comprenant un ou plusieurs sels de fer, sous réserve que le ou les sels de fer soient des sels autres que l'oxyde de fer ;
(b) d'application à la surface de la peau d'une composition de bronzage comprenant une quantité cosmétiquement efficace de la dihydroxyacétone comme agent de bronzage artificiel.

11. Procédé suivant la revendication 10, dans lequel la composition à base de sel de fer comprend n'importe quelle composition renfermant un ou plusieurs sels de fer qui est décrite dans l'une quelconque des revendications 1 à 10.

12. Procédé suivant l'une des revendications 10 et 11, dans lequel la composition de bronzage comprend n'importe quelle composition renfermant de la dihydroxyacétone qui est décrite dans l'une quelconque des revendications 1 à 10.

13. Procédé suivant l'une quelconque des revendications 10 à 12, dans lequel la composition de bronzage est appliquée après la composition à base de sels de fer.

14. Procédé suivant l'une quelconque des revendications 10 à 12, dans lequel la composition de bronzage et la composition à base de sels de fer sont appliquées pratiquement simultanément.

15. Procédé suivant la revendication 14, dans lequel la composition à base de sels de fer et la composition de bronzage forment conjointement une seule composition.
